# EUROPEAN PATENT APPLICATION

(11) **EP 4 578 388 A1**
(43) Date of publication of application: **02.07.2025**
(21) Application number: 24222712.2
(22) Date of filing: 23.12.2024
(51) Int. Cl.: A61B 5/287, A61B 5/00, A61B 5/0538

(54) **SYSTEMS AND METHODS OF INTRACARDIAC-ELECTROGRAM MEASUREMENT**

(30) Priority: 27.12.2023 US 202318396904
(71) Applicant: Biosense Webster (Israel) Ltd., 2066717 Yokneam (IL)
(72) Inventor: BUCHNIK, Yael, 2066717 Yokneam (IL); GLINER, Vadim, 2066717 Yokneam (IL)
(74) Representative: Carpmaels & Ransford LLP

(57) **Abstract**

Method and system to determine cardiac tissue activation signals are disclosed. The method provides to inter-alia determine a far-field component of Intracardiac Electrogram (IEGM) signal sensed by at least one electrode of a plurality of electrodes of a catheter. The method includes applying tissue proximity measurement to each respective electrode of a multitude of the catheter's electrodes to assess respective distances thereof respectively from a tissue surface; dynamically selecting, based on the respective distances, a subset of one or more electrodes of the multitude whose respective distances from the tissue surface are above a certain threshold; and determining a far-field component of the IEGM signal by averaging IEGM signal measurements from the electrodes of the subset whose respective distances are above the certain threshold.

## Description

### TECHNOLOGICAL FIELD

The present invention is in the field of signal processing of physiological signals and particularly relates to measurement of heart tissue activation signals such as intracardiac-electrograms (IEGM) used in electrocardiographic monitoring or mapping during medical procedures.

### BACKGROUND

A wide range of medical procedures involve placing probes, such as catheters, within a patient's body. One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Catheters are inserted into the heart chamber and optionally around the heart chamber during such procedures. In most procedures, multiple catheters are inserted into the patient. Catheters may include mapping, ablation, temperature sensing and image sensing catheters. Some catheters are dedicated for placement in specific parts of the anatomy, e.g., coronary sinus, esophagus, atrium, ventricle. The catheters have multiple electrical channels, some more than others depending on the number of sensors and electrodes included in each catheter. The number and type of catheters depends on the procedure and on the physician preferred workflow. During a procedure, the electrical activity of the heart is monitored/mapped from the catheter's electrodes and as well as optionally from body surface electrodes that are attached to the patient's skin.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** is a schematic illustration showing a catheter-based electrophysiology measurement/mapping system **10** according to an embodiment of the present invention;
**Fig. 2** is a schematic illustration of a catheter **14** having planar distal end assembly that is adapted for measurements of IEGM signals according to an embodiment of the present invention;
**Fig. 3** is a flow diagram illustrating a method **200** for measuring IEGM signals according to an embodiment of the present invention;
**Fig. 4** is a block diagram schematically illustrating the configuration of an IEGM signal measurement system **100** according to an embodiment of the present invention;

Like reference numerals are used in the figures to designate similar modules/elements of the present invention or elements/modules having like functionalities. Accordingly, unless otherwise specified, the description of modules/elements with reference to a certain embodiment of the invention should be understood to apply to all embodiments of the present invention, in which such module/element is incorporated.

### DESCRIPTION OF EMBODIMENTS OF THE INVENTION

There is a need in the art for a novel and inventive technique to accurately determine cardiac tissue activation signals.

Widely used conventional technologies for measuring or mapping the electrical properties of a heart tissue, often utilize catheters with volumetric distal end assemblies, such as basket or balloon catheters or otherwise catheters having multiple splines at their distal end that surrounding a certain volume. In such catheters, typically electrode(s) which are located on outer/external surface(s) of the distal end (e.g., on external surfaces of the catheter's splines), are operated to measure intracardiac-electrograms (IEGM) from tissue regions in contact therewith, and another electrode, which is typically located within the volume enclosed by the volumetric distal end and thus within the intracardiac cavity but remote from the intracardiac tissue, is operated measure a far-field components of the IEGM. Accordingly, the far-field components of the IEGM may than be used to suppress the far-field IEGM component in the IEGM signals obtained from the electrode(s) contacting the tissue (e.g. by subtracting far-field IEGM component from the IEGM signals obtained from the contacting electrode(s)), to thereby obtain near-field IEGM component(s) of the IEGM signals sensed by the contacting electrode(s). The development of the near-field component of IEGM signal sensed by a respective electrode contacting a tissue region overtime is indicative of the activation signal in the tissue region being in contact with the respective electrode.

Intra-cardiac catheters are typically disposable elements designated for single-use in an intracardiac operation. There is therefore a need in the art to reduce the fabrication costs of such catheters. As will be appreciated by the below description, one approach for reducing fabrication costs of catheters for measuring/mapping the electrical properties of a heart tissue, is to use catheters having planar distal end assembly. This significantly lowers the fabrication costs, as such planar distal end assembly can be formed by a flexible printed circuit board (PCB) with the electrodes printed thereon.

However, a challenge in using such catheters having the planar distal end assembly, for measuring or mapping the electrical properties of a heart tissue, arises from the fact that there is no specific location in the planar configuration of the distal end for placing an electrode designated for measuring the far-field IEGM signal component. Indeed, any of the electrodes on the planar distal end assembly may occasionally touch or not touch the heart tissue during operation, or be proximal thereto, and therefore the far-field IEGM signal component cannot be obtained reliably from any specific electrode.

The present invention thus provides a novel technique for measuring the far-field IEGM signal component in a manner suitable for use in catheters designated for measurement or mapping of electrical properties of a heart tissue. The invention may be for example used to determine far-field component of IEGM signals sensed by catheters, which do not have an electrode (e.g. dedicated electrode) that is specifically adapted/dedicated to sense the far-field comments of the IEGM signals (e.g. while without, or with substantially suppressed, sensing of "noise" such as near-field IEGM from its surroundings). A non-limiting example of such a catheter is the catheter described herein below which has a planar distal end assembly, but the invention is not limited to this specific type of catheter and may be used for determining the far-field component of IEGM signals sensed by other catheter types which do not have any specific electrode suited for far-field IEGM sensing. Accordingly, the present invention provides a solution to the above challenge, facilitating the accurate measurement of tissue activation signals, by such catheters.

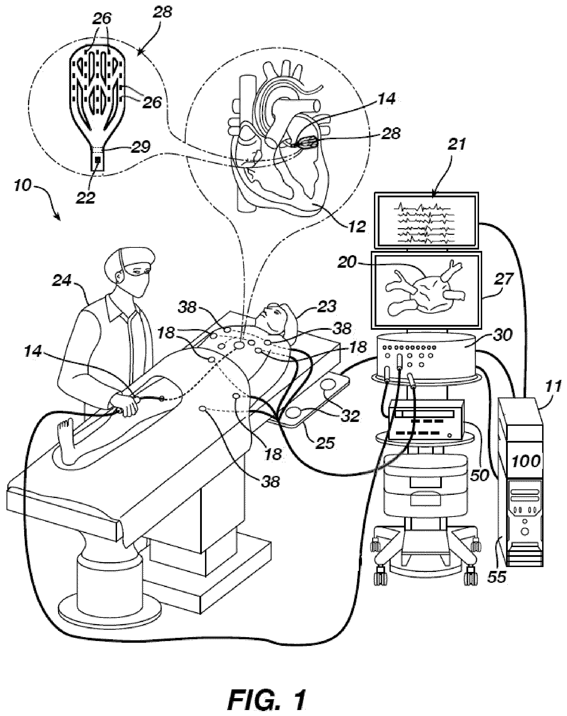

Reference is made to **Fig. 1** schematically exemplifying a catheter-based electrophysiology mapping system **10.** System **10** includes a catheter **14** for sensing cardiac tissue activation signals, or in other words Intracardiac Electrogram (IEGM) signals. Catheter **14** may be percutaneously inserted by a physician **24** through the patient's vascular system into a chamber or vascular structure of a heart **12.** Typically, a delivery sheath catheter is inserted into the left or right atrium, near a desired location in heart **12.** Thereafter, one or more catheters may be inserted into the delivery sheath catheter so as to arrive at the desired location in heart **12.** An example catheter **14,** that is configured for sensing IEGM is illustrated herein. Physician **24** may place a distal end assembly **28** of catheter **14** in contact with the heart wall for sensing a target site in heart **12.**

Catheter **14** is an exemplary catheter that includes multiple electrodes **26** at distal end portion **28** thereof for sensing IEGM signals from a heart tissue proximal thereto. Catheter **14** may additionally include a position sensor **29** embedded in or near distal end assembly **28** for tracking position and orientation of distal end assembly **28.** Optionally and preferably, position sensor **29** is a magnetic based position sensor including three magnetic coils for sensing three-dimensional (3D) location and/or orientation.

Magnetic based position sensor **29** may be operated together with a location pad **25,** which includes a plurality of magnetic coils **32** configured to generate magnetic fields in a predefined working volume. Real time position of distal end assembly **28** of catheter **14** may be tracked based on magnetic fields generated with location pad **25** and sensed by magnetic based position sensor **29.** System **10** optionally also includes one or more patches **38** positioned for skin contact on patient **23** to establish location reference for location pad **25.** Details of the magnetic based position sensing technology are described for example in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

A recorder **11** records and displays electrograms **21** captured with body surface electrocardiogram (ECG) electrodes **18** and intracardiac electrograms (IEGM) captured with electrodes **26** of catheter **14.** Recorder **11** may include pacing capability for pacing the heart rhythm and/or may be electrically connected to a standalone pacer.

In some embodiments the system **10** may be further adapted to perform tissue ablation. In such embodiments the system **10** may include an ablation energy generator **50** and a catheter dedicated for tissue ablation (not specifically shown). To this end, the system **10** may include a one or more catheters including catheter(s) dedicated for IEGM sensing and/or catheter(s) dedicated for ablating and/or catheter(s) dedicated for both IEGM and ablating. For ablation, physician **24** may similarly place a distal end of an ablation catheter in contact with a target site for ablating a tissue there. The ablation energy generator **50** is adapted to conduct ablative energy to one or more of electrodes at a distal end of the ablation catheter. Energy produced by ablation energy generator **50** may include, but is not limited to, radiofrequency (RF) energy or pulsed-field ablation (PFA) energy, including monopolar or bipolar high-voltage DC pulses as may be used to effect irreversible electroporation (IRE), or combinations thereof. In embodiments, the catheter **14** may also be configured and operable for tissue ablation, and may thus be adapted for both IEGM sensing and ablating.

Patient interface unit (PIU) **30** is an interface configured to establish electrical communication between medical devices, such as catheters and/or other electrophysiological equipment, and a workstation **55** for controlling operation of system **10.** The medical devices of system **10** may for example include electrophysiological equipment such as one or more catheters, location pad **25,** body surface ECG electrodes **18,** electrode patches **38,** ablation energy generator **50,** and recorder **11.** Optionally and preferably, PIU **30** additionally includes processing capability for implementing real-time computations of location of the catheters and for performing ECG/IEMG signalprocessing and/or calculations.

Workstation **55** includes one or more processors with memory and/or storage with appropriate operating software stored therein, and user interface capability. Workstation **55** may provide multiple functions, optionally including (1) modeling the endocardial anatomy in three-dimensions (3D) and rendering the model or anatomical map **20** for display on a display device **27,** (2) displaying on display device **27** (or other data) compiled from recorded electrograms **21** in representative visual indicia or imagery superimposed on the rendered anatomical map, (3) displaying real-time location and orientation of one or more catheters within the heart chamber, and (4) displaying, on display device **27,** sites of interest such as places where activations signals are measured/mapped, or ablation energy has been applied. One commercial product embodying elements of the system **10** is available as the CARTO^{™} 3 System, available from Biosense Webster, Inc., 31A Technology Drive, Irvine, CA 92618.

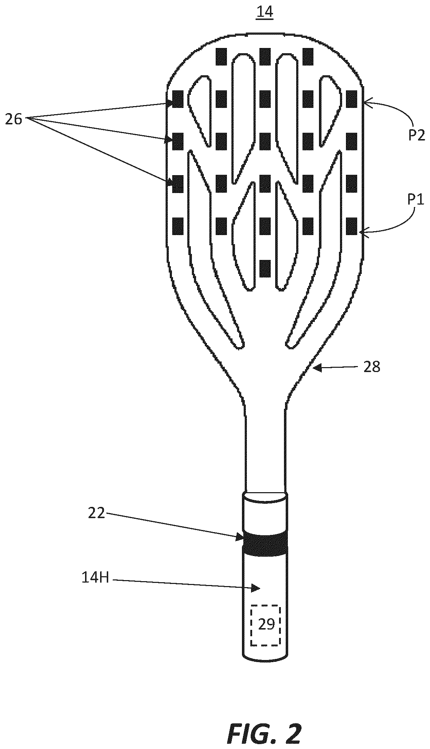

With reference to **Fig. 2****,** an example of a catheter **14** for IEGM sensing is schematically illustrated. In this example catheter **14** has a planar distal end **28** adapted for Intracardiac Electrogram (IEGM) sensing/mapping. More specifically, the catheter includes a shaft **14H** and a distal end assembly **28** that is connected at one end of the shaft **14H,** whereby the distal end assembly **28** of the catheter in this example has a planar configuration, and includes a plurality of electrodes **26** suitable of IEGM sensing. The plurality of electrodes **26** generally include a first and second pluralities of the electrodes arranged respectively on opposite surfaces **P1** and **P2** of the planar configuration of the distal end assembly **28** (in the figure only the electrodes **26** from one side of the planar distal end assembly **28** are shown).

The shaft **14H** includes a reference electrode **22** arranged on the shaft **14H** near the distal end assembly **28.** As described in more details below, the reference electrode **22** facilitates measurement of distances between the electrodes **26** and the heart tissue and thereby enables to assess a far-field component of the IEGM signal sensed thereby according to the technique of the present invention as described in more detail below. As will be appreciated by those versed in the art, in order to measure cardiac tissue activation signal at region of interest of a heart tissue, a far field component of the IEGM signals, should generally be measured (e.g. by electrode sufficiently distanced from the heart tissue yet still within the blood pool of the heart) The far-field component should then be subtracted/suppressed from the IEGM signals measured by each electrode touching the heart tissue at the region of interest, to thereby obtain a near-field component of the IEGM signals at that region of interest, which overtime is indicative of the cardiac tissue activation signal at the tissue region of interest. However, in some catheters, such as catheter **14** whose distal end assembly **28** is planar, there may be no specific electrode(s) dedicated for measuring the far-field component of the IEGM signals, and more specifically there is no specific electrode that is a priory maintained distanced from the tissue wall of the heart such that it can accurately measure the far field component of the IEGM signals sensed by the electrodes **26.** For example, during operation with the planar distal end assembly **28** of catheter **14,** all or some of the electrodes located on one surface (e.g. **P1**) of the planar distal end assembly **28** may touch the tissue of the heart wall, while all or some of the electrodes **26** located on the opposite surface (e.g. **P2**) may not touch the heart tissue. As the distal end assembly **28** in this catheter example **14** is planar (i.e. not volumetric as for instance in basket or ballon catheter types), there is no specific location on the distal end assembly **28** at which an electrode can be placed to verifiably not touch the heart tissue. Thus, in such type of catheter, in which there is no specific electrode designated for sensing the far-field IEGM, there is a need to dynamically identify and select, during the operation, electrode(s) of the plurality of electrodes **26,** which are not touching the heart tissue and are sufficiently distanced therefrom, and use the signals from those selected electrode(s) to determine the far-field EGM signal. To achieve that, the reference electrode **22** is furnished on the catheter **14** and adapted/dedicated for implementation of tissue proximity sensing/measurements based on impedance between each one of electrodes **26** and the reference electrode **22,** to thereby assess the distances of one or more of the electrodes **26** from the heart tissue.

In this non-limiting example, the reference electrode **22** is arranged on the shaft **14H** of the catheter **14,** and is adapted to facilitate the tissue proximity measurements. Then IEGM signals, which are acquired/measured by a subset of the electrodes **26** that were identified as being sufficiently distanced from the tissue, are used to determine/assess the far-field component of the IEGM signals that is sensed by one or more of the electrodes **26** that are in contact with the tissue. In order to facilitate the tissue proximity measurement(s), the reference electrode **22** in this example is arranged on the shaft **14H** of the catheter in a manner such that during intracardiac operation of the catheter **14** within the heart it contacts body fluids (for example the blood when the catheter is inserted through patient's vascular system), while typically maintained distanced from, and not in contact with, the tissue/heart-tissue. This arrangement enables using the reference electrode **22** for the impedance-based tissue proximity measurements, by which the impedance between it and each respective electrode of electrodes **26** can be used to assess the distance of the respective electrode from the heart tissue. Preferably, in some embodiments the reference electrode **22** is configured with a ring like shape (e.g. surrounds the shaft **14H**).

Indeed, although the reference electrode **22,** in this case is on the shaft **14H,** is arranged such as it typically does not contact the tissue, it may still not be suitable to provide accurate measurement of the far-field IEGM component. One reason for that may be that the reference electrode is typically relatively large and therefore it may capture both near-field and far-field components in case it brought close to the tissue wall. One reason for that may be that as the reference electrode **22** is placed on the shaft **14H** (and is typically relatively large) it is generally too close to the tissue (e.g. tissue of the vascular system through which the catheter may be inserted, thus not suited for accurate sensing of the far-field signals), and thus may capture both far-field component of the IEGM signal as well as near-field components thereof from the tissue region proximal thereto. Another reason is that in some cases, where the catheter **14** is delivered via a delivery sheath, the reference electrode **22** which is located on the shaft **14H,** may remain within the delivery sheath and thereby substantially masked from sensing the far-field component of the IEGM signal.

Nonetheless, according to embodiments of the invention, the reference electrode **22** may be used to assess the distances of one or more of the electrodes **26** from the heart tissue and thereby facilitate the identification and dynamic selections of subsets of electrodes **26,** which are sufficiently distant from the tissue, and by which the far-field signal can be measured with accuracy. As described in more detail below, the distance measurements of the electrodes **26,** also referred to herein as *tissue proximity measurement(s),* and or tissue proximity index/indices, may be performed by measuring the impedances of respective electrode(s) **26** whose tissue proximity is to be assessed (e.g. impedances between each respective electrode and the reference electrode **22**) and determine the tissue proximities of one or more of the electrodes **26** based on their impedances. Example for tissue proximity measurements/techniques which may be implemented according to the present invention to assess the tissue proximities of one or more of the electrodes **26** are disclosed for example in U.S. Patent Application Publication No. 2021/0177504 which is incorporated herein by reference.

Optionally, the shaft **14H** also includes a position sensor **29** which is typically embedded at or near the catheter's distal end assembly **28** to enable tracking position of the distal end assembly **28** (e.g. and the electrodes **26** thereon) by system **100,** and thereby enable special mapping of the activation signals sensed by electrodes **26** or some of them.

Typically, the catheter **14** is implemented as a disposable catheter. In some embodiments, the planar distal end assembly **28** of catheter **14** is configured with a flexible printed circuit board (PCB) and with the first and second electrode pluralities of the electrodes **26** furnished/fabricated on opposite sides/surfaces, **P1** and **P2** of the flexible PCB. This inter-*alia* provides for cost effective fabrication of the catheter **14,** reducing its production costs of and thus also costs of medical procedures, such as epicardial procedures, in which it may be utilized.

Turning back to **Fig. 1****,** as indicated above the electrophysiological equipment, such as the surface ECG device **38** which includes a plurality of ECG electrode patches attached externally to the patient's body/skin, as well as other electrophysiological equipment such as additional catheters (not specifically shown) that are connected to the PIU **30,** which facilitate electrical communication between these electrophysiological equipment and the workstation **55.** The PIU **30** may for example include signal processors adapted inter-*alia* to apply analog to digital conversion (sampling) to the signals received from the electrophysiological equipment connected thereto, and/or pack these signals in data packets and communicate them to the workstation **55** and/or to recorder **11.**

According to the present invention the system **10** includes a IEGM signal measurement system **100** (subsystem of system **10**) capable of at least determining a far-field component of IEGM signals sensed by electrode(s) **26** of a catheter, such as catheter **14,** which may have no specific electrode designated for measuring the far field IEGM signal component. The IEGM signal measurement system **100** may further be adapted to determine near-field component(s) of IEGM signal(s) measured by one or more of the electrodes **26** of the catheter, and optionally thereby determine the activations signals **AS** in the tissue regions proximal to the one or more electrodes **26.** In various implementation the system **100** is implemented as a subsystem of system **10,** and components of the system **100** may be included or implemented in any one or more of the: workstation **55,** PIU **30** and recorder **11** of system **10** or distributed between them (e.g. implemented by one or more processors and/or signal processors of those sub-systems).

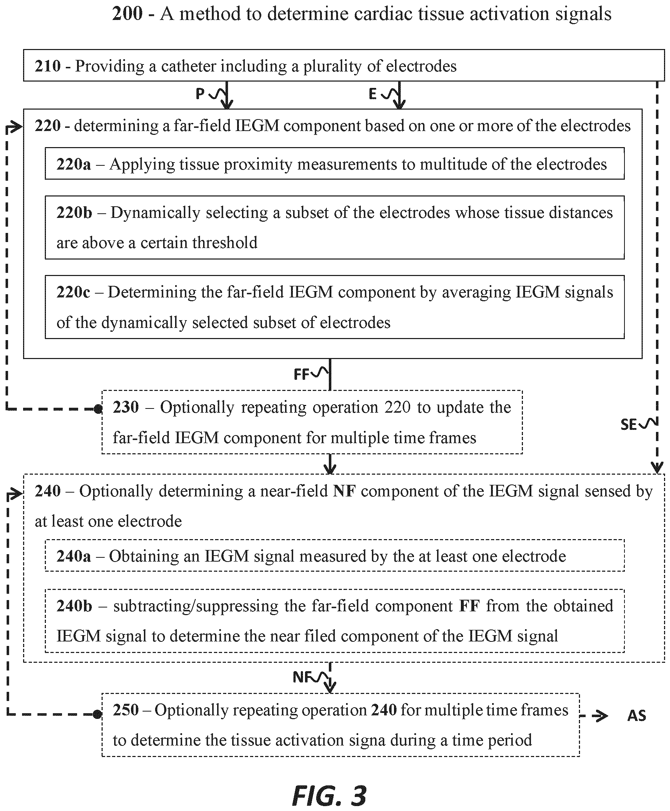

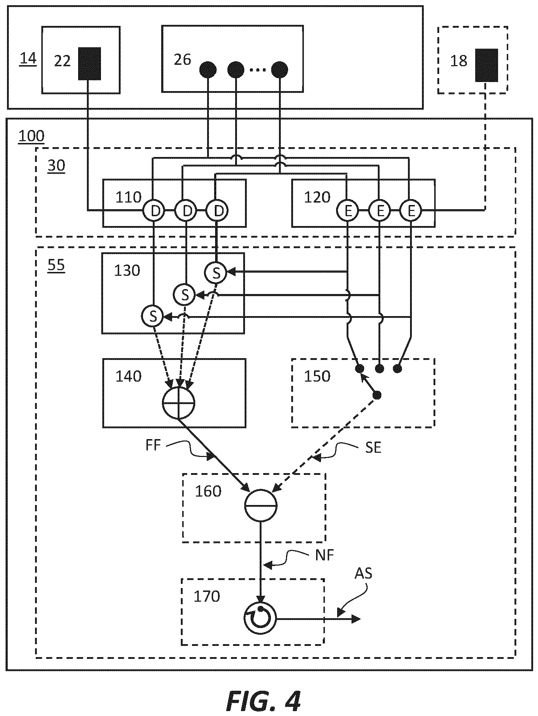

Reference is now made together to **Figs. 3** and **4** exemplifying the technique for measuring far-field IEGM components, and optionally also near field components and/or tissue activation signals, according to embodiments of the present invention. **Fig. 3** is a flow diagram of a method **200** to determine far-field IEGM component, and optionally based on the far-field IEGM component also near-field IEGM components and/or cardiac tissue activation signals. **Fig. 4** is a block diagram exemplifying an embodiment of an IEGM signal measurement system **100** that implements the method **200.**

In operation **210** of method **200,** a catheter, such as catheter **14,** is provided, having a plurality of electrodes **26** arranged at a distal end assembly **28** thereof, in a manner suitable for sensing IEGM signals from tissue regions in contact therewith respectively. The electrodes **26** may for example be arranged at outer surfaces of the distal end assembly **28** of the catheter **14.** Optionally the catheter also includes a reference electrode **22** as described above. Alternatively, in some implementation the reference electrode **22** may not necessarily reside on the catheter **14** and may be arranged in a different manner to contacts body fluids while maintained distanced from be body/heart tissue.

Operation **220** of method **200,** is carried out to determine a far-field component **FF** of the IEGM signals **E** sensed by the electrode(s) **26** of the catheter **14.** To achieve that, in operation **220a** a tissue proximity measurement is applied to each respective electrode of a multitude of the electrodes **26** to assess respective distances **D** of electrodes of the multitude from the surface of the tissue (e.g., heart tissue). The multitude of electrodes, whose tissue proximities are assessed in this case may include one or more of the plurality of electrodes **26** at the distal end assembly **28** of the catheter **14,** and typically preferably more than one electrode. In some embodiments the tissue proximity measurements are implemented for example by a technique similar to that disclosed in U.S. Patent Application Publication No. 2021/0177504.

For instance, the tissue proximity measurements, may be implemented by tissue proximity processor/processing **110** of system **100.** For example, the tissue proximity processing **110** may be carried out by a signal processor which is associated with the system and connected to the multitude of the electrodes **26** and the reference electrode **22.** The signal processor implementing the tissue proximity processing **110** may deliver current including one or more frequency components in between each respective of the electrode of the multitude of the electrodes **26** and the reference electrode **22,** and measure the respective impedance between them (e.g. optionally the measured impedance(s) per each frequency component in the delivered current). As the tissue has typically significantly different impedance than body fluids/blood, the respective impedance measured in this way, between each respective electrode of and the reference electrode **22,** provides indication **D** of the distance between the respective electrode and the tissue.

More specifically, as the impedance readings are generally very sensitive to tissue proximity and may vary from patient to patient, in some embodiments the relation between impedances and respective distances **D** of the electrodes, may be inferred dynamically during the medical procedure. In such embodiments, optionally in operation **220a** (e.g. in at least one iteration of this operation), the impedance values from each electrode whose impedance is being measured, may be stored by the system **100.** Then, based on properties of the distribution of the measured impedance values (e.g. for example based on the maximal and minimal impedances measured), a relation between measured impedance of an electrode and its distance **D** from the tissue, and/or an impedance threshold indicative of whether the electrode contacts the tissue or not, may be determined in real time during the medical procedure. Accordingly, based on this relation or impedance-threshold, the distances **D** of the respective electrodes may be assessed in **220a** from their measured impedances.

Thus, as illustrated the none-limiting example of **Fig. 4** data/signals indicative of respective distances **D** of a multitude of the electrodes **26** is determined and provided in this manner by the tissue proximity processing **110.**

In parallel, however not necessarily concurrently with the tissue proximity measurement operation **220a** but typically following it, IEGM signal measurements, referenced **E** in **Fig. 4****,** are obtained from one or more of the plurality of electrodes **26** respectively. The IEGM signal measurements **E** may be implemented for example by IEGM signal measurement processor/processing **120** of system **100** as illustrated in **Fig. 4****.** The IEGM signals **E** are typically measured for least some of the electrodes **26,** which may include the electrodes **26** whose respective tissue distances **D** are assessed in operation **220a** to be above a certain distance threshold D_{TH}, as well as optionally for one or more electrodes of electrodes **26** whose near-field IEGM signal component is to be inferred in the optional operation **240** described below. Alternatively in some embodiments the IEGM signals **E** may be measured for all or a predetermined set of the electrodes **26.** The IEGM signals **E** may be measured for the electrodes according to any suitable technique as will be appreciated by those versed in the art. For instance, in some implementations the IEGM signal **E** of a respective electrode of electrodes **26** may be measured by carrying out a unipolar IEGM signal measurement as known in the art, to monitor/measure the voltage (e.g., during a time frame) between the respective electrode and another electrode(s), which may be for instance one or more of the body surface ECG electrodes **18** and/or one or more of the electrode patches **38** of system **10** described above.

In some none-limiting embodiments, the tissue proximity processing **110** and the IEGM signal measurement processing **120** may be implemented by a signal processor, which may be a part of the PIU **30** of system **10** described above. The signal processor, e.g. PIU **30,** may be for example in signal communication with the electrodes **26,** as well as optionally with the reference electrode **22** (used for the tissue proximity processing), and optionally also with the another electrode e.g. **18,** which may be used for unipolar IEGM signal measurements. The signal processor may implement various signal processing capabilities, for instance impedance and/or voltage measurements between electrodes coupled thereto, signal/noise filtering, analog-to-digital conversion and/or other signal processing as will be appreciated by those versed in the art. As will be described in more detail below the tissue proximity processing **110** and the IEGM signal measurement processing **120** may be performed repeatedly during successive time frames (e.g. each being in the order of for example of 50 milliseconds (ms)), in order to assess the tissue proximities **D** for multitudes of the electrodes **26** and the IEGM signal measurements **E** for some or all of them as described above, for each successive time frame. In case analog-to-digital conversion is implemented, each such time frame may include for instance 50 samples, e.g. assuming a sampling rate in the order of 1 kHz is applied.

In order to determine/assess the far-field IEGM component **FF** sensed (e.g. commonly) by the electrodes **26,** operation **220** of method **200** further includes the sub-operations **220b** and **220c.** In operation **220b,** subset of electrodes **26** whose respective tissue distances/proximities **D** were assessed in operation **220a** to be above a certain distance threshold D_{TH}, are selected (e.g. dynamically selected per each time frame) for further processing of their IEGM signals **E** to determine based thereon the (common) far-field IEGM component **FF** in the IEGM signals. The minimal distance threshold D_{TH}, above which the electrodes are selected is generally such that the IEGM signals sensed thereby is expected to be mostly composed of the far-field component **FF** and to lesser degree of near-field IEGM components **NF.** For instance, the distance threshold D_{TH} may be selected as at least D_{TH} > 5 millimeters.

In operation **220c** the far-field component **FF** of the IEGM signal is determined (e.g. for the respective time frame) by averaging (or otherwise aggregating by a different aggregation scheme) the IEGM signal measurements **E** from the respective electrodes **26** of the subset selected in **220b** whose respective distances **D** from the tissue surface are above the minimal distance threshold D_{TH}. The aggregation/averaging of the signals from the plurality of electrodes provide for suppressing/averaging-out noise components (e.g. near-field signal residues which may still be weakly sensed by the electrodes of the subset in spite of their relative distance from the tissue) to thereby reliably obtain the far-field component **FF** substantially "clean" from noise.

For some time-frames, operation **220b** may not yield any selected electrodes. For example, in case the reference electrode **22** happens to touch a tissue near it during a timeframe, the distance measurements **D** obtained in operation **220a** for all/any of the electrodes **26** may provide values below the threshold D_{TH} for all the electrodes **26.** Therefore, in operation **220c,** in cases where a number of the electrodes identified as sufficient tissue distance is below a certain predetermined minimal number of electrodes (e.g. the minimal number being at least one and typically more than one), the previous value of the far-field IEGM signal **FF** measured for a preceding timeframe may be used in the consecutive timeframe. This generally does not introduce significant artifacts to the measured far-field IEGM signal **FF** since these cases are typically rare due to the location/configuration of the reference electrode **22,** and also because the timeframes are typically of duration smaller than a characteristic time interval for change in the far-field component **FF** of the IEGM signal.

For example, in operation **220a** impedances of all, or of a plurality of, electrodes **26** from both sides of the planar distal end assembly **28,** may be measured with reference to electrode **22.** In case all the impedances being measured in **220a** are indicative of the respective electrodes being in contact with the tissue wall (e.g., being relatively high impedances), then in this case, in operation **220b** the system **100** may assert the that the origin of the high impedance is that the reference electrode **22** is touching the cavity/tissue wall (since it would be typically impossible for pluralities of electrodes on both sides of the planar distal end assembly **28** to simultaneously touch the tissue wall), and no electrode will be dynamically selected as being distant from the tissue. Therefore, in this case, in operation **220c** the previous value of the far-field IEGM signal **FF** will be provided as representative of the far-field IEGM signal **FF** also for the current timeframe.

With reference to the system **100** illustrated in **Fig. 4****,** the dynamic selection **220b** of the subset of electrodes whose distances **D** from the tissue are sufficient (e.g. above threshold D_{TH}) may be performed by a signal selector utility **130** of the system **100.** The aggregation/averaging **220c** of the signals from the subset selected by signal selector utility **130** may be performed by a signal aggregation utility **140** of the system **100.** As will be appreciated by those versed in the art the signal selector utility **130** and/or the signal aggregation utility **140** may be implemented by signal processing (e.g. as part of the **PIU 30** of system **10**) and/or by a computerized system/processor, such as the workstation **55** or the recorder **11** illustrated in **Fig. 1****.** Signal selector utility **130** may include selectors **S** (e.g. implemented as signal switches, and/or by digital/computerized selection process(s) or by other means as will be appreciated by those versed in the art). Accordingly, the signal aggregation utility **140** may also be implemented by analog, digital or computerized summation/averaging or other form of aggregation.

In view of the above, in operation **220** the far-field **FF** component of the IEGM signal may be determined based on the IEGM signals **E** measured by the electrodes **26,** while generally obviating a need to have a dedicated electrode arranged in the catheter **14** for sensing the intra-cardiac far-field IEGM component **FF.**

As indicated above the operation **220** may be carried out for assessing determining the far-field **FF** component of the IEGM signal per respective time frames (successive/consecutive time frames), at which the IEGM signals **E** are measured by the electrodes **26.** Accordingly, as illustrated in the optional operation **230** of method **200,** the operation **220** may be repeated for multiple successive or consecutive time frames, in order to update the value of the far-field IEGM component **FF** for multiple time-frames over a desired time duration of a medical operation at which the **IEGM** signals are to be monitored. As indicated above, for timeframes (e.g. generally rare) where the field IEGM component **FF** cannot be assessed (e.g. in case reference electrode **22** touches the tissue), the far-field IEGM component **FF** assessed for the respectively preceding time frame may be used. To this end, system **100** may include a buffer (data or signal buffer not specifically shown) holding the value of the last updated field IEGM component **FF** in the preceding timeframe, to enable this value to be used in such cases).

Optionally, method **200** further includes operation **240** for assessing a near field components **NF** of an IEGM signal sensed by certain one or more electrodes of interest **SE** of the electrodes **26** (typically some of the electrodes **26** which are in contact with the cardiac tissue). The set of the certain electrodes for which the near-field component **NF** is to be determined, may in various implementations be automatically selected by the system **10** (e.g. in accordance with cardiac electrical activity being mapped or measured thereby, possibly based on the tissue regions of interest being mapped/measured and the position of the catheter **14** as may be determined by position sensor **29** ), and/or it may be selected in some implementations by physician **24** operating the system **10.**

Operation **240** optionally includes sub-operation **240a,** in which IEGM signal(s) **E** measured by the certain one or more electrodes of interest **SE** are obtained (at least one electrode is illustrated in the none-limiting example of **Figs. 3** and **4**). In sub-operation **240b,** the far-field component **FF,** as determined by operation **220** above, is also obtained. The far-field component **FF** is used to processes the IEGM signal(s) **E** of each of the one or more electrodes of interest **SE** to determine/assess the respective near-field components **NF,** of IEGM signal(s) **E** sensed thereby respectively. Generally, the near-field **NF** component of each electrode of interest **SE** is determined by suppressing or subtracting the far-field component **FF** obtained by operation **220** from the IEGM signal **E** measured by the electrode **SE.** This is because the IEGM signal **E** measured by each electrode of interest **SE** is composed of both the near-field **NF** component which is sensed by the electrode from the tissue in its vicinity and the far-field component far-field component **FF** which is typically commonly sensed by the electrodes **26.** In this regard, as will be appreciated by those versed in the art of signal manipulation/processing, the subtraction/suppression may be performed with proper weighting of the FF signal according to the amplitude, in which it may be included in the IEGM signal of the respective electrode of interest **SE** (e.g. a proper weight may for example be determined based on correlation between the **FF** components and the respective IEGM signal).

To implement the optional operation **240,** system **100** as illustrated in **Fig. 4****,** may for example include an optional electrode selector **150,** which may be adapted to provide the IEGM signals measured by the certain one or more electrodes of interest **SE,** and signal suppression utility/filter **160** which is adapted to receive the far-field IEGM component **FF** and the IEGM signal measured by each respective electrode of interest **SE** and to suppress/subtract the former from the latter in order to yield the near-field component **NF** of the IEGM signal component of the respective electrode of interest **SE.** As will be appreciated by those versed in the art the electrode selector **150** and/or the signal suppression utility/filter **160** may for example be implemented by analog or by digital means, for example as a part of a signal processor (e.g. by PIU **30**) or by a computerized system (e.g. by work station **55**) illustrated in **Fig. 1****.**

Generally, operation **240,** may be performed per each respective time frame of interest at which the near field components **NF** of certain electrodes of interest **SE** are to be assessed. To this end, as will be appreciated from the above description, the operation **240** may be performed in synchronization with operation **220,** such that the far-field component **FF** obtained for a respective time frame is suppressed from the IEGM signal **E** obtained from each electrode of interest **SE,** for the corresponding/same time frame to thereby yield the near-field signal **NF** sensed by that electrode **SE** during that time frame.

Optionally method **200** may also further include operation **250** by which operation **240** is repeated for multiple time frames to determine/record the tissue activation signal **AS** sensed by each electrode of interest **SE** from the tissue in its vicinity. The tissue activation signal **AS** is used herein to designate an aggregation/accumulation of the near-field signal **NF** components measured by each respective measured electrode of interest **SE** in each time frame for over a time duration one of multiple time frames. Operation **250** may for example be performed by a recorder **170** of system **100** illustrated in **Fig. 4****,** which may for example be part of recorder **11** of system **10** which records the tissue activation signals of the electrodes of interest **SE.**

To this end, system **100** and method **200** described above exemplify implementations of the technique of the present invention to assess far-field signal components **FF** of IEGM signals **E** sensed by a plurality of electrodes **26** on a distal end assembly of a catheter, such as catheter **14,** which may not have a suitable electrode dedicated for sensing intra-cardiac far-field IEGM signal **FF.** For clarity the system **100** is illustrated in **Fig. 4** with separate designation of multiple components thereof performing different functions/operations of method **100.** However as will be appreciated by those versed in the art, the system **100** may generally implemented with a different set of components, and for example may be implemented by a general signal processor and/or a general-purpose computerized system with software and/or hardware adapted to perform the operations of method **200** described above. Additionally, as indicated above, systems and methods implementing the invention may be adapted to determine the far-field components of IEGM signals measured in one or more timeframes and optionally thereby also determine the near-field components of the IEGM signal for said timeframes, and further thereby optionally determine the tissue activation signals based on the nearfield components assessed for the multiple time frames.

### Examples

Example 1. A method **220** to determine cardiac tissue activation signals, the method includes:
**I.** providing a catheter **14** including a plurality of electrodes **26** arranged at a distal end assembly **28** thereof;
**II.** determining **220** a far-field component **FF** of Intracardiac Electrogram (IEGM) signal **E** sensed by at least one electrode of the plurality of the electrodes **26** by carrying out the following:
   **(a)** applying tissue proximity measurement **220a** to each respective electrode of a multitude of the electrodes **26** to assess respective distances **D** of the multitude of electrodes from a tissue surface;
   **(b)** dynamically selecting **220b,** based on said respective distances **D,** a subset of one or more electrodes of said multitude of the electrodes **26** whose respective distances from the tissue surface are above a certain threshold; and
   **(c)** determining said far-field component **FF** by averaging IEGM signal measurements **E** from the respective electrodes of said subset whose respective distances **D** from the tissue surface are above said certain threshold.

Example 2. The method **200** according to example 1, further includes:
**III.** assessing **240** a near-field component **NF** of the IEGM signal sensed by said at least one electrode by subtracting said far-field component from an IEGM signal measurement obtained from said at least one electrode.

Example 3. The method **200** according to example 2, further includes repeating (**230, 250**) the operations II and III to determine development of the near-field **NF** component of the EGM signal **E** over time and thereby obtaining a cardiac tissue activation signal of the tissue near said at least one electrode.

Example 4. The method **200** according to example 1, wherein applying the tissue proximity measurement (**220a**) to the respective electrode includes applying an excitation current through the respective electrode and measuring an impedance of the electrode and thereby assessing the tissue proximity **D** based on said impedance.

Example 5. The method **200** according to example 4, wherein the impedance is measured between the respective electrode and a reference electrode **22** arranged near an end of a shaft **14H** of the catheter **14** proximal to the distal end assembly **28,** such that it typically remains spaced from the tissue during operation of the catheter **14.**

Example 6. The method **200** according to example 1, wherein the far-field component **FF** of IEGM signal **E** is repeatedly updated (**230**) by repeating operation II (**220**).

Example 7. The method **200** according to example 6, wherein updates of the far-field component **FF** of IEGM signal **E** are skipped in repetitions (**230**) of operation II in which a number of the electrodes identified by the dynamic selection (**220b**) as having the respective distances **D** from the tissue surface above the certain threshold D_{TH}, is below a certain predetermined minimal number of electrodes. For example, the predetermined minimal number of electrodes with distance **D** above the certain threshold D_{TH}, that below this number the far-field component **FF** is not updated and its preceding value remains being one or more electrodes.

Example 8. The method **200** according to example 7, wherein applying said tissue proximity measurement to said respective electrode (**220a**) includes measuring an impedance between the respective electrode and a reference electrode **22** that is arranged on the catheter **14,** such that it typically remains spaced from the tissue; and wherein in repetitions (**230**) in which the number of the electrodes is below the certain predetermined minimal number, the reference electrode **22** is assumed to be in contact with the tissue, therefore skipping the updates of the far-field component of EGM signal in those repetitions.

Example 9. The method **200** according to example 1, wherein the distal end assembly **28** of the catheter **14** has a planar configuration, and the plurality of EGM electrodes **26** includes a first and second pluralities of the EGM electrodes arranged respectively on opposite surfaces, **P1** and **P2,** of the distal end assembly **28.**

Example 10. The method **200** according to example 1, adapted to enable the assessment of the far-field component **FF** of the EGM signals **E** while without the catheter **14** having a dedicated electrode arranged in its distal end assembly **28** for sensing the far-field component **FF.**

Example 11. The method **200** according to example 9, wherein the distal end assembly of the catheter **14** includes a flexible printed circuit board (PCB) with the first and second electrode pluralities at opposite sides of the PCB.

Example 12. A system **100** to determine cardiac tissue activation signals, the system **100** being connectable to a catheter **14** having a plurality of electrodes **26** arranged at a distal end assembly **28** thereof;
wherein the system **100** includes one or more processors connectable for signal communication with electrodes **26** of the plurality; and
wherein the one or more processors are adapted to determine (**220**) a far-field component **FF** of Intracardiac Electrogram (IEGM) signal **E** sensed by at least one electrode of the plurality of the electrodes **26** by carrying out the following:
   **(a)** applying (**220a**) tissue proximity measurement to each respective electrode of a multitude of the electrodes **26** to assess respective distances **D** of the multitude of electrodes from a tissue surface;
   **(b)** dynamically selecting (**220b**), based on the respective distances **D,** a subset of one or more electrodes of the multitude of the electrodes whose respective distances **D** from the tissue surface are above a certain threshold D_{TH}; and
   **(c)** obtaining IEGM signal measurements **E** from the respective electrodes of said subset whose respective distances **D** from the tissue surface are above said certain threshold D_{TH}; and
   **(d)** determining (**220c**) the far-field component **FF** as an average of the IEGM signal measurements **E** obtained from the respective electrodes of the subset whose respective distances **D** from the tissue surface are above said certain threshold D_{TH}.

Example 13. The system **100** according to example 12, wherein the one or more processors are adapted to further assess a near-field component **NF** of the IEGM signal **E** sensed by at least one of the electrodes **26** by subtracting the far-field component **FF** from the IEGM signal measurement obtained from the at least one electrode.

Example 14. The system **100** according to example 13, wherein the one or more processors are adapted to determine cardiac tissue activation signal **AS** in a tissue near said at least one electrode by repeatedly (**230, 250**) determining (**230, 250**) the far-field and near-field components, **FF** and **NF,** and thereby determining development of the near-field component **NF** of the IEGM signal over time, whereby the development being indicative of the cardiac tissue activation signal **AS.**

Example 15. The system **100** according to example 12, wherein the one or more processors are adapted to apply the tissue proximity measurement (**220a**) to the respective electrode by delivering an excitation current through the respective electrode and measuring an impedance of the electrode, to thereby assess the tissue proximity based on the impedance.

Example 16. The system **100** according to example 15, wherein the delivery of the excitation current is made between the respective electrode and a reference electrode **22** arranged near an end of the shaft **14H** proximal to the distal end assembly **28** of the catheter **14,** such that it typically remains spaced from the tissue.

Example 17. The system **100** according to example 12 wherein the one or more processor are adapted to repeatedly update (**230**) the far-field component of IEGM signal by repeating method operations **220a** to **220c;** and wherein updates of the far-field component of IEGM signal are skipped in repetitions of these in which a number of the electrodes identified by the dynamic selection operation **220b** as having the respective distances **D** from the tissue surface above the certain threshold D_{TH}, is below a certain predetermined minimal number of electrodes (the mini number may be one or in some cases more than one to facilitate improved measurement/assessment of the far-field components by averaging the signals of several electrodes).

Example 18. A catheter **14** for Intracardiac Electrogram (IEGM) mapping. The catheter **14** includes a shaft **14H** and a distal end assembly **28** connected at one end of the shaft **14H;** wherein the distal end assembly **28** has a planar configuration and includes a plurality of electrodes **26** including a first and second pluralities of the electrodes arranged respectively on opposite surfaces, **P1** and **P2,** of the planar configuration of the distal end assembly **28,** and a reference electrode **22** arranged on the shaft **14H** in vicinity of the distal end assembly **28** to enable conduction of respective tissue proximity measurement for one or more respective electrodes of the plurality by measuring an impedances between the reference electrode **22** and the respective electrodes **26.**

Example 19. The catheter **14** according to example 18, wherein at least one of the following:
- the distal end assembly **28** includes a flexible printed circuit board (PCB) with the first and second electrode pluralities at opposite sides of the PCB;
- the reference electrode **22** is positioned on said shaft such during intracardiac operation of the catheter within a heart, the reference electrode **28** is typically distanced from a tissue of the heart, while being in contact with blood to thereby facilitate the tissue proximity measurement based on said impedance; and
- the reference electrode has a ring like shape.

Example 20. The catheter **14** according to example 18 configured as a disposable catheter.

Example 21. A method to determine cardiac tissue activation signals, the method comprises:
I. providing a catheter including a plurality of electrodes arranged at a distal end assembly thereof;
II. determining a far-field component of Intracardiac Electrogram (IEGM) signal sensed by at least one electrode of the plurality of the electrodes by carrying out the following:
   (a) applying tissue proximity measurement to each respective electrode of a multitude of the electrodes to assess respective distances of the multitude of electrodes from a tissue surface;
   (b) dynamically selecting, based on said respective distances, a subset of one or more electrodes of said multitude of the electrodes whose respective distances from the tissue surface are above a certain threshold; and
   (c) determining said far-field component by averaging IEGM signal measurements from the respective electrodes of said subset whose respective distances from the tissue surface are above said certain threshold.

Example 22. The method according to example 21 further comprising:
I. assessing a near-field component of the IEGM signal sensed by said at least one electrode by subtracting said far-field component from an (concurrent) IEGM signal measurement obtained from said at least one electrode.

Example 23. The method according to example 22 comprising repeating the operation II and III to determine development of said near-field component of the EGM signal over time and thereby obtaining a cardiac tissue activation signal of the tissue near said at least one electrode.

Example 24. The method according to example 21 wherein applying said tissue proximity measurement to said respective electrode in **(a)** comprises applying an excitation current through said respective electrode and measuring an impedance of said electrode and thereby assessing said tissue proximity based on said impedance.

Example 25. The method according to example 24 wherein said impedance is measured between said respective electrode and a reference electrode arranged near an end of a shaft of the catheter proximal to said distal end assembly, such that it typically remains spaced from the tissue during operation of the catheter.

Example 26. The method according to example 21 wherein said far-field component of IEGM signal is repeatedly updated by repeating operation II.

Example 27. The method according to example 26 wherein updates of the far-field component of IEGM signal are skipped in repetitions of operation II in which a number of the electrodes identified by said dynamic selection (b) as having said respective distances from the tissue surface above said certain threshold, is below a certain predetermined minimal number of electrodes.

Example 28. The method according to example 27 wherein applying said tissue proximity measurement to said respective electrode in (a) comprises measuring an impedance between said respective electrode and a reference electrode being arranged to typically remain spaced from the tissue; and wherein in repetitions in which said number of the electrodes is below said certain predetermined minimal number, the reference electrode is assessed to be in contact with the tissue, therefore skipping the updates of the far-field component of EGM signal in those repetitions.

Example 29. The method of example 21 wherein said distal end assembly of the catheter has a planar configuration and said plurality of EGM electrodes comprise a first and second pluralities of the EGM electrodes arranged respectively on opposite surfaces at said distal end assembly of the catheter.

Example 30. The method according to example 21 adapted to enable the assessment of said far-field component of the EGM signals while without said catheter having a dedicated electrode arranged in its distal end assembly for sensing the far-field component.

Example 31. The method according to example 29 wherein said distal end assembly of the catheter comprises a flexible printed circuit board (PCB) with said first and second electrode pluralities at opposite sides of the PCB.

## Claims

1. A system to determine cardiac tissue activation signals,
the system being connectable to a catheter having a plurality of electrodes arranged at a distal end assembly thereof;
wherein the system comprises one or more processors connectable for signal communication with electrodes of said plurality; and
wherein said one or more processors are adapted to determine a far-field component of Intracardiac Electrogram (IEGM) signal sensed by at least one electrode of the plurality of the electrodes by carrying out the following:
(a) applying tissue proximity measurement to each respective electrode of a multitude of the electrodes to assess respective distances of the multitude of electrodes from a tissue surface;
(b) dynamically selecting, based on said respective distances, a subset of one or more electrodes of said multitude of the electrodes whose respective distances from the tissue surface are above a certain threshold; and
(c) obtaining IEGM signal measurements from the respective electrodes of said subset whose respective distances from the tissue surface are above said certain threshold; and
(d) determining said far-field component as an average of the IEGM signal measurements obtained from the respective electrodes of said subset whose respective distances from the tissue surface are above said certain threshold.

2. The system according to claim 1 wherein said one or more processors are adapted to further assess a near-field component of the IEGM signal sensed by at least one of said electrodes by subtracting said far-field component from an IEGM signal measurement obtained from said at least one electrode.

3. The system according to claim 2 wherein said one or more processors are adapted to determine cardiac tissue activation signal in a tissue near said at least one electrode by repeatedly determining said far-field and near-field components and thereby determining development of said near-field component of the IEGM signal over time whereby said development being indicative of the cardiac tissue activation signal.

4. The system according to claim 1 wherein said one or more processor are adapted to apply said tissue proximity measurement to said respective electrode in **(a)** by delivering an excitation current through said respective electrode and measuring an impedance of said electrode to thereby assess said tissue proximity based on said impedance.

5. The system according to claim 4 wherein the delivery of said excitation current impedance is made between said respective electrode and a reference electrode arranged near an end of the shaft proximal to the distal end assembly of the catheter, such that it typically remains spaced from the tissue.

6. The system according to claim 1 wherein said one or more processor are adapted to repeatedly update said far-field component of IEGM signal by repeating operations (a) to (d); and wherein updates of the far-field component of IEGM signal are skipped in repetitions of operation (a) to (d) in which a number of the electrodes identified by said dynamic selection (b) as having said respective distances from the tissue surface above said certain threshold, is below a certain predetermined minimal number of electrodes.

7. A catheter for Intracardiac Electrogram (IEGM) mapping comprising a shaft and a distal end assembly connected at one end of the shaft wherein the distal end assembly of the catheter has a planar configuration and includes a plurality of electrodes including a first and second pluralities of the electrodes arranged respectively on opposite surfaces of the planar configuration of the distal end assembly, and a reference electrode arranged on said shaft in vicinity of said distal end assembly to enable conduction of respective tissue proximity measurement for one or more respective electrodes of said plurality by measuring an impedances between said reference electrode and the respective electrodes.

8. The catheter according to claim 7 wherein at least one of the following:
said distal end assembly comprises a flexible printed circuit board (PCB) with said first and second electrode pluralities at opposite sides of the PCB;
said reference electrode is positioned on said shaft such during intracardiac operation of the catheter within a heart, the reference electrode is typically distanced from a tissue of the heart, while being in contact with blood to thereby facilitate the tissue proximity measurement based on said impedance; and
said reference electrode has a ring like shape.

9. The catheter according to claim 7 configured as a disposable catheter.
